(19) Europäisches Patentamt

European Patent Office

Office européen des brevets

(11) EP 0 908 515 A2

(12) **EUROPEAN PATENT APPLICATION**

(43) Date of publication:
14.04.1999 Bulletin 1999/15

(21) Application number: 98304691.3

(22) Date of filing: 15.06.1998

(51) Int Cl.⁶: **C12N 15/12**, C07K 14/47, A61K 38/17, C12Q 1/68, G01N 33/68, G01N 33/50, C07K 16/18

(84) Designated Contracting States:
**AT BE CH CY DE DK ES FI FR GB GR IE IT LI LU MC NL PT SE**
Designated Extension States:
**AL LT LV MK RO SI**

(30) Priority: 16.09.1997 EP 97307187

(71) Applicant: **SMITHKLINE BEECHAM PLC**
**Brentford, Middlesex TW8 9EP (GB)**

(72) Inventor: **Southan, Christopher D.,**
**SmithKline Beecham Pharm.**
**Harlow, Essex, CM19 5AW (GB)**

(74) Representative:
**Connell, Anthony Christopher et al**
**SmithKline Beecham plc**
**Corporate Intellectual Property,**
**Two New Horizons Court**
**Brentford, Middlesex TW8 9EP (GB)**

(54) **Pancreatic polypeptide**

(57) Novel pancreatic polypeptides and polynucleotides and methods for producing such polypeptides by recombinant techniques are disclosed. Also disclosed are methods for utilizing novel pancreatic polypeptides and polynucleotides in the design of protocols for the treatment of food intake-related disorders such as obesity, anorexia and bulimia; asthma; Parkinson's disease; acute heart failure; hypotension; hypertension; urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain, among others, and diagnostic assays for such conditions.

EP 0 908 515 A2

## Description

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to the neuropeptide family, hereinafter referred to as novel pancreatic polypeptide. The invention also relates to inhibiting or activating the action of such polynucleotides and polypeptides.

### BACKGROUND OF THE INVENTION

It is well established that neuropeptides are synthesised by proteolytic processing of a large preprohormone precursor protein. Peptides within preprohormones are typically flanked by pairs of basic residues, for example, Lys-Arg (KR), Arg-Arg (RR), Lys-Lys (KK) or Arg-Lys (RK). Neuropeptides may also undergo post-translational modifications that include disulphide bond formation, glycosylation, COOH-terminal alpha-amidation, phosphorylation and sulphation (V. Y. H. Hook et al., FASEB J, 8: 1269-1278, 1994 and refs therein). It is not uncommon for the preprohormone processing to give rise to more than one neuropeptide, for example, the precursor of human pancreatic polypeptideand pancreatic isopeptide (Boel et al. EMBO J. 3(4) 909-912, 1984). The synthesised neuropeptides exert their biological action by interacting with the membrane protein gene superfamily of G-protein coupled receptors (Lefkowitz, Nature, 1991, 351:353-354). G-protein coupled receptors have been characterized as including these seven conserved hydrophobic stretches of about 20 to 30 amino acids, connecting at least eight divergent hydrophilic loops. The G-protein family of coupled receptors includes dopamine receptors which bind to neuroleptic drugs used for treating psychotic, neurological and other disorders. Other examples of members of this family include, but are not limited to, pancreatic polypeptide, calcitonin, adrenergic, endothelin, cAMP, adenosine, muscarinic, acetylcholine, serotonin, histamine, thrombin, kinin, follicle stimulating hormone, opsins, endothelial differentiation gene-1, rhodopsins, odorant, and cytomegalovirus receptors. Over the past 15 years, nearly 350 therapeutic agents targeting 7 transmembrane (7 TM) receptors or their ligands have been successfully introduced onto the market.

Antibiotic activity has also been reported for Neuropeptide Y (NPY) and polypeptide P (PYY) (Vouldoukis et al FEBS Lett, 380(3):237-240, 1996).

There is however a continuing need for identification and characterization of further members of the neuropeptide family which can play a role in preventing, ameliorating or correcting dysfunctions or diseases, including, but not limited to, food intake-related disorders such as obesity, anorexia and bulimia; asthma; Parkinson's disease; acute heart failure; hypotension; hypertension; urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain.

### SUMMARY OF THE INVENTION

In one aspect, the invention relates to novel pancreatic polypeptides and recombinant materials and methods for their production. Another aspect of the invention relates to methods for using such novel pancreatic polypeptides and polynucleotides. Such uses include the treatment of food intake-related disorders such as obesity, anorexia and bulimia; asthma; Parkinson's disease; acute heart failure; hypotension; hypertension; urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain, among others. A further use includes the use of the novel pancreatic polypeptides as an antibacterial peptide. In still another aspect, the invention relates to methods to identify agonists and antagonists using the materials provided by the invention, and treating conditions associated with novel pancreatic polypeptide imbalance with the identified compounds. Yet another aspect of the invention relates to diagnostic assays for detecting diseases associated with inappropriate novel pancreatic polypeptide activity or levels.

### DESCRIPTION OF THE INVENTION

#### Definitions

The following definitions are provided to facilitate understanding of certain terms used frequently herein.

"novel pancreatic polypeptide" refers, among others, generally to a polypeptide having the amino acid sequence set forth in SEQ ID NO:2 or an allelic variant thereof.

"novel pancreatic polypeptide activity or novel pancreatic polypeptide activity" or "biological activity of the novel pancreatic polypeptide or novel pancreatic polypeptide" refers to the metabolic or physiologic function of said novel pancreatic polypeptide including similar activities or improved activities or these activities with decreased undesirable side-effects. Also included are antigenic and immunogenic activities of said novel pancreatic polypeptide.

"novel pancreatic polypeptide gene" refers to a polynucleotide having the nucleotide sequence set forth in SEQ ID NO: 1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single-and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NYAcad Sci* (1992) 663 :48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in (Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., SIAM *J. Applied Math., 48*: 1073 (1988). Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., Nucleic Acids Research *12(1):* 387 (1984)), BLASTP, BLASTN, and FASTA (Atschul, S.F. et al., J. Molec. Biol. *215:* 403-410 (1990). The BLAST X program is publicly available from NCBI and other sources (BLAST Manual, Altschul, S., *et al.,* NCBI NLM NIH Bethesda, MD 20894; Altschul, S., *et al.,* J. Mol. Biol. *215*: 403-410 (1990). The well known Smith Waterman algorithm may also be used to determine identity.

Preferred parameters for polypeptide sequence comparison include the following:

1) Algorithm: Needleman and Wunsch, J. Mol Biol. 48: 443-453 (1970)

Comparison matrix: BLOSSUM62 from Hentikoffand Hentikoff, Proc. Natl. Acad. Sci. USA. 89:10915-10919 (1992)

Gap Penalty: 12

Gap Length Penalty: 4

A program useful with these parameters is publicly available as the "gap" program from Genetics Computer Group, Madison WI. The aforementioned parameters are the default parameters for polypeptide comparisons (along with no penalty for end gaps).

Preferred parameters for polynucleotide comparison include the following:

1) Algorithm: Needleman and Wunsch, J. Mol Biol. 48 : 443-453 (1970)

Comparison matrix: matches = +10, mismatch = 0

Gap Penalty: 50

Gap Length Penalty: 3

A program useful with these parameters is publicly available as the "gap" program from Genetics Computer Group, Madison WI. The aforementioned parameters are the default parameters for polynucleotide comparisons.

By way of example, a polynucleotide sequence of the present invention may be identical to the reference sequence of SEQ ID NO: 1, that is be 100% identical, or it may include up to a certain integer number of nucleotide alterations as compared to the reference sequence. Such alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence. The number of nucleotide alterations is determined by multiplying the total number of nucleotides in SEQ ID NO: 1 by the numerical percent of the respective percent identity(divided by 100) and subtracting that product from said total number of nucleotides in SEQ ID NO: 1, or:

$$n_n \leq x_n - (x_n \bullet y),$$

wherein $n_n$ is the number of nucleotide alterations, $x_n$ is the total number of nucleotides in SEQ ID NO:1, and $y$ is, for instance, 0.70 for 70%, 0.80 for 80%, 0.85 for 85%, 0.90 for 90%, 0.95 for 95%,etc., and wherein any non-integer product of $x_n$ and $y$ is rounded down to the nearest integer prior to subtracting it from $x_n$. Alterations of a polynucleotide sequence encoding the polypeptide of SEQ ID NO:2 may create nonsense, missense or frameshift mutations in this coding sequence and thereby alter the polypeptide encoded by the polynucleotide following such alterations.

Similarly, a polypeptide sequence of the present invention may be identical to the reference sequence of SEQ ID NO:2, that is be 100% identical, or it may include up to a certain integer number of amino acid alterations as compared to the reference sequence such that the % identity is less than 100%. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 by the numerical percent of the respective percent identity(divided by 100) and then subtracting that product from said total number of amino acids in SEQ ID NO:2, or:

$$n_a \leq x_a - (x_a \bullet y),$$

wherein $n_a$ is the number of amino acid alterations, $x_a$ is the total number of amino acids in SEQ ID NO:2, and $y$ is, for instance 0.70 for 70%, 0.80 for 80%, 0.85 for 85% etc., and wherein any non-integer product of $x_a$ and $y$ is rounded down to the nearest integer prior to subtracting it from $x_a$.

**Polypeptides of the Invention**

In one aspect, the present invention relates to novel pancreatic polypeptides (or novel pancreatic proteins). The novel pancreatic polypeptides include the polypeptide of SEQ ID NO:2; as well as polypeptides comprising the amino acid sequence of SEQ ID NO: 2; and polypeptides comprising the amino acid sequence which have at least 85% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within novel pancreatic polypeptides are polypeptides having the amino acid sequence which have at least 85% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% are highly preferred. Preferably novel pancreatic polypeptide exhibit at least one biological activity of novel pancreatic polypeptide.

The novel pancreatic polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the novel pancreatic polypeptides are also included in the invention. A fragment is a polypeptide having an amino acid sequence that entirely is the same as part, but not all, of the amino acid sequence of the aforementioned novel pancreatic polypeptides. As with novel pancreatic polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40,41-60, 61-80, 81-100, and 101 to the end of novel pancreatic polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of novel pancreatic polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate novel pancreatic polypeptide activity, including those with a similar activity or an improved activity, or with a decreased undesirable activity. Also included are those that are antigenic or immunogenic in an animal, especially in a human.

Preferably, all of these polypeptide fragments retain the biological activity of the novel pancreatic polypeptide, including antigenic activity. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The novel pancreatic polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

**Polynucleotides of the Invention**

Another aspect of the invention relates to novel pancreatic polypeptide polynucleotides. Novel pancreatic polypeptide polynucleotides include isolated polynucleotides which encode the novel pancreatic polypeptides and fragments, and polynucleotides closely related thereto. More specifically, novel pancreatic polypeptide polynucleotide of the invention include a polynucleotide comprising the nucleotide sequence contained in SEQ ID NO: 1 encoding a novel pancreatic polypeptide of SEQ ID NO: 2, and polynucleotide having the particular sequence of SEQ ID NO: 1. novel pancreatic polypeptide polynucleotides further include a polynucleotide comprising a nucleotide sequence that has at least 95% identity over its entire length to a nucleotide sequence encoding the novel pancreatic polypeptide of SEQ ID NO:2, and a polynucleotide comprising a nucleotide sequence that is at least 95% identical to of SEQ ID NO: 1 over its entire length. Furthermore, polynucleotides with at least 97% identity are highly preferred and those with at least 98-99% identity are most highly preferred, with at least 99% identity being the most preferred. Also included under novel pancreatic polypeptide polynucleotides are a nucleotide sequence which has sufficient identity to a nucleotide sequence contained in SEQ ID NO: 1 to hybridize under conditions useable for amplification or for use as a probe or marker. The invention also provides polynucleotides which are complementary to such novel pancreatic polypeptide polynucleotides.

Novel pancreatic polypeptide of the invention is structurally related to other proteins of the neuropeptide family, as shown by the results of sequencing the cDNA of Table 1 (SEQ ID NO:1) encoding human novel pancreatic polypeptide. The cDNA sequence of SEQ ID NO: 1 contains an open reading frame (nucleotide number 1 to 284) encoding a polypeptide of 95 amino acids of SEQ ID NO:2. Amino acid sequence of Table 2 (SEQ ID NO:2) has about 83% identity (using BESTFIT) in 95 amino acid residues with human pancreatic polypeptide (Boel et al. EMBO J. 3(4) 909-912, 1984). Nucleotide sequence of Table 1 (SEQ ID NO:1) has about 91% identity (using BESTFIT) in 284 nucleotide residues with human pancreatic polypeptide (Boel et al. EMBO J. 3(4) 909-912, 1984). Thus novel pancreatic polypeptides and polynucleotides of the present invention are expected to have, inter alia, similar biological functions/properties to their homologous polypeptides and polynucleotides.

## Table 1[a]

```
ATGGCTGCCGCATGCCGCTGCCTCTCCCTCCTGCTCCTGTCCACCTGTGTGGCTCTGTTGCT
GCAGCCACTGCTGGGTGCCCGGGGAGCCCCGTTGGAGCCATTGTACCCAGGGGACAATACCA
CACCGGAGCAGATGGCCCAGTACACAGCTGAGCTCCGTAGATACATCAACATGCTGACCAGG
CATAGGTATGGAGAAAGAGACAAAGAGGACACGCTGGCCTTCTCAGAGTGGGGGTCTTCCCA
```

```
TGCTGCTGTCCCCAGAGAGCTCAGCCCGCTGGACTTG
```

[a] A nucleotide sequence of a human novel pancreatic polypeptide - SEQ ID NO: 1.

## Table 2[b]

```
MAAACRCLSLLLLSTCVALLLQPLLGARGAPLEPLYPGDNTTPEQMAQYTAEL
RRYINMLTRHRYGERDKEDTLAFSEWGSSHAAVPRELSPLDL
```

[b] An amino acid sequence of a human novel pancreatic polypeptide - SEQ ID NO: 2.

One polynucleotide of the present invention encoding novel pancreatic polypeptide may be obtained using standard cloning and screening, from a cDNA library derived from mRNA in cells of human foetal spleen using the expressed sequence tag (EST) analysis (Adams, M.D., *et al. Science* (1991) 252:1651-1656; Adams, M.D. *et al., Nature,* (1992) *355*:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding novel pancreatic polypeptide of SEQ ID NO:2 may be identical to the polypeptide encoding sequence contained in Table 1 (nucleotide number 1 to 284 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of novel pancreatic polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain noncoding 5' and 3' sequences, such as transcribed, non-translated sequences, splicing and polyadenylation signals, ribosome binding sites and sequences that stabilize mRNA.

Further preferred embodiments are polynucleotides encoding novel pancreatic polypeptide variants comprise the amino acid sequence novel pancreatic polypeptide of Table 2 (SEQ ID NO:2) in which several, 5-10,1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 80%, and preferably at least 90%, and more preferably at least 95%, yet even more preferably 97-99% identity between the sequences.

Polynucleotides of the invention, which are identical or sufficiently identical to a nucleotide sequence contained in SEQ ID NO: 1 or a fragment thereof, may be used as hybridization probes for cDNA and genomic DNA, to isolate full-length cDNAs and genomic clones encoding novel pancreatic polypeptide and to isolate cDNA and genomic clones of other genes (including genes encoding homologs and orthologs from species other than human) that have a high sequence similarity to the novel pancreatic polypeptide gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding novel pancreatic polypeptide, including homologs and orthologs from species other than human, comprises the steps of screening an appropriate library under stingent hybridization conditions with a labeled probe having the SEQ ID NO: 1 or a fragment thereof; and isolating full-length cDNA and genomic clones containing said polynucleotide sequence. Thus in another aspect, novel pancreatic polypeptide polynucleotides of the present invention further include a nucleotide sequence comprising a nucleotide sequence that hybridize under stringent condition to a nucleotide sequence having SEQ ID NO: 1 or a fragment thereof. Also included with novel pancreatic polypeptides are polypeptide comprising amino acid sequence encoded by nucleotide sequence obtained by the above hybridization condition. Such hybridization techniques are well known to those of skill in the art. Stringent hybridization conditions are as defined above or, alternatively, conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

## Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING : A LABORATORY MANUAL,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the novel pancreatic polypeptide is to be expressed for use in screening assays, generally, it is preferred that the polypeptide be produced at the surface of the cell. In this event, the cells may be harvested prior to use in the screening assay. If novel pancreatic polypeptide is secreted into the medium, the medium can be recovered in order to recover and purify the polypeptide; if produced intracellularly, the cells must first be lysed before the polypeptide is recovered. novel pancreatic polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

**Diagnostic Assays**

This invention also relates to the use of novel pancreatic polypeptide polynucleotides for use as diagnostic reagents. Detection of a mutated form of novel pancreatic polypeptide gene associated with a dysfunction will provide a diagnostic tool that can add to or define a diagnosis of a disease or susceptibility to a disease which results from under-expression, over-expression or altered expression of novel pancreatic polypeptide. Individuals carrying mutations in the novel pancreatic polypeptide gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled novel pancreatic polypeptide nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e. g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising novel pancreatic polypeptide nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to food intake-related disorders such as obesity, anorexia and bulimia; asthma; Parkinson's disease; acute heart failure; hypotension; hypertension; urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain through detection of mutation in the novel pancreatic polypeptide gene by the methods described.

In addition, food intake-related disorders such as obesity, anorexia and bulimia; asthma; Parkinson's disease; acute heart failure; hypotension; hypertension; urinary retention; osteoporosis; angina pectoris; myocardial infarction;

ulcers; asthma; allergies; benign prostatic hypertrophy; and psychotic and neurological disorders, including anxiety, schizophrenia, manic depression, delirium, dementia, severe mental retardation and dyskinesias; cancer and pain, can be diagnosed by methods comprising determining from a sample derived from a subject an abnormally decreased or increased level of novel pancreatic polypeptide or novel pancreatic polypeptide mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an novel pancreatic polypeptide, in a sample derived from a host are well-known to those of skill in the art. Such assay methods include radioimmunoassays, competitive-binding assays, Western Blot analysis and ELISA assays.

Thus in another aspect, the present invention relates to a diagonostic kit for a disease or suspectability to a disease, particularly food intake-related disorders such as obesity, anorexia and bulimia; asthma; Parkinson's disease; acute heart failure; hypotension; hypertension; urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain, which comprises:

> (a) a novel pancreatic polypeptide polynucleotide, preferably the nucleotide sequence of SEQ ID NO: 1, or a fragment thereof ;
> (b) a nucleotide sequence complementary to that of (a);
> (c) a novel pancreatic polypeptide, preferably the polypeptide of SEQ ID NO: 2, or a fragment thereof; or
> (d) an antibody to a novel pancreatic polypeptide, preferably to the polypeptide of SEQ ID NO: 2. It will be appreciated that in any such kit, (a), (b), (c) or (d) may comprise a substantial component.

**Chromosome Assays**

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

The novel pancreatic polypeptide has been localised to chromosone 17 at 17 q 22-23.

**Antibodies**

The polypeptides of the invention or their fragments or analogs thereof, or cells expressing them can also be used as immunogens to produce antibodies immunospecific for the novel pancreatic polypeptides. The term "immunospecific" means that the antibodies have substantiall greater affinity for the polypeptides of the invention than their affinity for other related polypeptides in the prior art.

Antibodies generated against the novel pancreatic polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against novel pancreatic polypeptides may also be employed to treat food intake-related disorders such as obesity, anorexia and bulimia; asthma; Parkinson's disease; acute heart failure; hypotension; hypertension;

urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain, among others.

**Vaccines**

Another aspect of the invention relates to a method for inducing an immunological response in a mammal which comprises inoculating the mammal with novel pancreatic polypeptide, or a fragment thereof, adequate to produce antibody and/or T cell immune response to protect said animal from food intake-related disorders such as obesity, anorexia and bulimia; asthma; Parkinson's disease; acute heart failure; hypotension; hypertension; urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain, among others. Yet another aspect of the invention relates to a method of inducing immunological response in a mammal which comprises, delivering novel pancreatic polypeptide via a vector directing expression of novel pancreatic polypeptide polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a novel pancreatic polypeptide wherein the composition comprises a novel pancreatic polypeptide or novel pancreatic polypeptide gene. The vaccine formulation may further comprise a suitable carrier. Since novel pancreatic polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

**Screening Assays**

The novel pancreatic polypeptide of the present invention may be employed in a screening process for compounds which activate (agonists) or inhibit activation of (antagonists, or otherwise called inhibitors) the novel pancreatic polypeptide of the present invention. Thus, polypeptides of the invention may also be used to assess identify agonist or antagonists from, for example, cells, cell-free preparations, chemical libraries, and natural product mixtures. These agonists or antagonists may be natural or modified substrates, ligands, receptors, enzymes, etc., as the case may be, of the polypeptide of the present invention; or may be structural or functional mimetics of the polypeptide of the present invention. See Coligan *et al., Current Protocols in Immunology* 1(2):Chapter 5 (1991).

Novel pancreatic polypeptides are responsible for many biological functions, including many pathologies. Accordingly, it is desirous to find compounds and drugs which stimulate novel pancreatic polypeptide on the one hand and which can inhibit the function of novel pancreatic polypeptide on the other hand. In general, agonists are employed for therapeutic and prophylactic purposes for such conditions as food intake-related disorders such as obesity, anorexia and bulimia; asthma; Parkinson's disease; acute heart failure; hypotension; hypertension; urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain. Antagonists may be employed for a variety of therapeutic and prophylactic purposes for such conditions as food intake-related disorders such as obesity, anorexia and bulimia; asthma; Parkinson's disease; acute heart failure; hypotension; hypertension; urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain.

In general, such screening procedures may involve using appropriate cells which express the novel pancreatic polypeptide or respond to novel pancreatic polypeptide of the present invention. Such cells include cells from mammals, yeast, *Drosophila* or *E. coli*. Cells which express the novel pancreatic polypeptide (or cell membrane containing the expressed polypeptide) or respond to novel pancreatic polypeptide polypeptide are then contacted with a test compound to observe binding, or stimulation or inhibition of a functional response. The ability of the cells which were contacted with the candidate compounds is compared with the same cells which were not contacted for novel pancreatic polypeptide activity.

The assays may simply test binding of a candidate compound wherein adherence to the cells bearing the novel pancreatic polypeptide is detected by means of a label directly or indirectly associated with the candidate compound or in an assay involving competition with a labeled competitor. Further, these assays may test whether the candidate compound results in a signal generated by activation of the novel pancreatic polypeptide, using detection systems appropriate to the cells bearing the novel pancreatic polypeptide. Inhibitors of activation are generally assayed in the presence of a known agonist and the effect on activation by the agonist by the presence of the candidate compound is observed.

Further, the assays may simply comprise the steps of mixing a candidate compound with a solution containing a novel pancreatic polypeptide polypeptide to form a mixture, measuring novel pancreatic polypeptide activity in the mixture, and comparing the novel pancreatic polypeptide activity of the mixture to a standard.

The novel pancreatic polypeptide cDNA, protein and antibodies to the protein may also be used to configure assays for detecting the effect of added compounds on the production of novel pancreatic polypeptide mRNA and protein in cells. For example, an ELISA may be constructed for measuring secreted or cell associated levels of novel pancreatic polypeptide protein using monoclonal and polyclonal antibodies by standard methods known in the art, and this can be used to discover agents which may inhibit or enhance the production of novel pancreatic polypeptide (also called antagonist or agonist, respectively) from suitably manipulated cells or tissues.

The novel pancreatic polypeptide protein may be used to identify membrane bound or soluble receptors, if any, through standard receptor binding techniques known in the art. These include, but are not limited to, ligand binding and crosslinking assays in which the novel pancreatic polypeptide is labeled with a radioactive isotope (eg 125I), chemically modified (eg biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. In addition to being used for purification and cloning of the receptor, these binding assays can be used to identify agonists and antagonists of novel pancreatic polypeptide which compete with the binding of novel pancreatic polypeptide to its receptors, if any. Standard methods for conducting screening assays are well understood in the art.

Examples of potential novel pancreatic polypeptide antagonists include antibodies or, in some cases, oligonucleotides or proteins which are closely related to the ligands, substrates, receptors, enzymes, etc., as the case may be, of the novel pancreatic polypeptide, e.g., a fragment of the ligands, substrates, receptors, enzymes, etc.; or small molecules which bind to the polypetide of the present invention but do not elicit a response, so that the activity of the polypeptide is prevented.

Thus in another aspect, the present invention relates to a screening kit for identifying agonists, antagonists, ligands, receptors, substrates, enzymes, etc. for novel pancreatic polypeptides; or compounds which decrease or enhance the production of novel pancreatic polypeptides, which comprises:

(a) a novel pancreatic polypeptide, preferably that of SEQ ID NO:2;
(b) a recombinant cell expressing a novel pancreatic polypeptide, preferably that of SEQ ID NO:2;
(c) a cell membrane expressing a novel pancreatic polypeptide; preferably that of SEQ ID NO: 2; or
(d) antibody to a novel pancreatic polypeptide, preferably that of SEQ ID NO: 2.

It will be appreciated that in any such kit, (a), (b), (c) or (d) may comprise a substantial component.

## Prophylactic and Therapeutic Methods

This invention provides methods of treating abnormal conditions such as, food intake-related disorders such as obesity, anorexia and bulimia; asthma; Parkinson's disease; acute heart failure; hypotension; hypertension; urinary retention; osteoporosis; angina pectoris; myocardial infarction; ulcers; asthma; allergies; benign prostatic hypertrophy; and psychotic and neurological disorders, including anxiety, schizophrenia, manic depression, delirium, dementia, severe mental retardation and dyskinesias; cancer and pain, related to both an excess of and insufficient amounts of novel pancreatic polypeptide activity.

If the activity of novel pancreatic polypeptide polypeptide is in excess, several approaches are available. One approach comprises administering to a subject an inhibitor compound (antagonist) as hereinabove described along with a pharmaceutically acceptable carrier in an amount effective to inhibit the function of the novel pancreatic polypeptide, such as, for example, by blocking the binding of ligands, substrates, receptors, enzymes, etc., or by inhibiting a second signal, and thereby alleviating the abnormal condition. In another approach, soluble forms of novel pancreatic polypeptides still capable of binding the ligand, substrate, enzymes, receptors, etc. in competition with endogenous novel pancreatic polypeptide may be administered. Typical embodiments of such competitors comprise fragments of the novel pancreatic polypeptide.

In still another approach, expression of the gene encoding endogenous novel pancreatic polypeptide can be inhibited using expression blocking techniques. Known such techniques involve the use of antisense sequences, either internally generated or separately administered. See, for example, O'Connor, *J Neurochem* (1991) 56:560 in <u>Oligodeoxynucleotides as Antisense Inhibitors of Gene Expression,</u> CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo.*

For treating abnormal conditions related to an under-expression of novel pancreatic polypeptide and its activity, several approaches are also available. One approach comprises administering to a subject a therapeutically effective amount of a compound which activates novel pancreatic polypeptide, i.e., an agonist as described above, in combination with a pharmaceutically acceptable carrier, to thereby alleviate the abnormal condition. Alternatively, gene therapy may be employed to effect the endogenous production of novel pancreatic polypeptide by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer a therapeutic amount of novel pancreatic polypeptides in combination with a suitable pharmaceutical carrier.

**Formulation and Administration**

Peptides, such as the soluble form of novel pancreatic polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo*, and for example, by the use of a retroviral plasmid vector. The cells are then introduced into the subject.

All publications, including but not limited to patents and patent applications, cited in this specification are herein incorporated by reference as if each individual publication were specifically and individually indicated to be incorporated by reference herein as though fully set forth.

## Claims

1. An isolated polynucleotide comprising a nucleotide sequence that has at least 85% identity over its entire length to a nucleotide sequence encoding the novel pancreatic polypeptide of SEQ ID NO:2; or a nucleotide sequence complementary to said isolated polynucleotide.

2. The polynucleotide of claim 1 wherein said polynucleotide comprises the nucleotide sequence contained in SEQ ID NO: 1 encoding the novel pancreatic polypeptide of SEQ ID NO2.

3. An isolated polynucleotide comprising a nucleotide sequence that is at least 95% identical to that of SEQ ID NO: 1 over its entire length.

4. The polynucleotide of claim 3 which is polynucleotide of SEQ ID NO: 1.

5. A DNA or RNA molecule comprising an expression system, wherein said expression system is capable of producing a novel pancreatic polypeptide comprising an amino acid sequence, which has at least 85% identity with the polypeptide of SEQ ID NO:2 when said expression system is present in a compatible host cell.

6. A host cell comprising the expression system of claim 5.

7. A process for producing a novel pancreatic polypeptide comprising culturing a host of claim 6 under conditions sufficient for the production of said polypeptide and recovering the polypeptide from the culture.

8. A process for producing a cell which produces a novel pancreatic polypeptide thereof comprising transforming or transfecting a host cell with the expression system of claim 6 such that the host cell, under appropriate culture conditions, produces a novel pancreatic polypeptide.

9. A novel pancreatic polypeptide comprising an amino acid sequence which is at least 85% identical to the amino acid sequence of SEQ ID NO:2 over its entire length.

10. The polypeptide of claim 9 which comprises the amino acid sequence of SEQ ID NO:2.

11. An antibody immunospecific for the novel pancreatic polypeptide of claim 9.

12. A method for the treatment of a subject in need of enhanced activity or expression of novel pancreatic polypeptide of claim 9 comprising:

    (a) administering to the subject a therapeutically effective amount of an agonist to said polypeptide; and/or
    (b) providing to the subject an isolated polynucleotide comprising a nucleotide sequence that has at least 85% identity to a nucleotide sequence encoding the novel pancreatic polypeptide of SEQ ID NO:2 over its entire length; or a nucleotide sequence complementary to said nucleotide sequence in a form so as to effect production of said polypeptide activity *in vivo*.

13. A method for the treatment of a subject having need to inhibit activity or expression of novel pancreatic polypeptide of claim 9 comprising:

(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate , or receptor.

14. A process for diagnosing a disease or a susceptibility to a disease in a subject related to expression or activity of novel pancreatic polypeptide of claim 9 in a subject comprising:

(a) determining the presence or absence of a mutation in the nucleotide sequence encoding said novel pancreatic polypeptide in the genome of said subject; and/or
(b) analyzing for the presence or amount of the novel pancreatic polypeptide expression in a sample derived from said subject.

15. A method for identifying compounds which inhibit (antagonize) or agonize the novel pancreatic polypeptide of claim 9 which comprises:

(a) contacting a candidate compound with cells which express the novel pancreatic polypeptide (or cell membrane expressing novel pancreatic polypeptide) or respond to novel pancreatic polypeptide; and
(b) observing the binding, or stimulation or inhibition of a functional response; or comparing the ability of the cells (or cell membrane) which were contacted with the candidate compounds with the same cells which were not contacted for novel pancreatic polypeptide activity.

16. An agonist or an antagonist identified by the method of claim 1.

17. A recombinant host cell produced by a method of Claim 8 or a membrane thereof expressing a novel pancreatic polypeptide.

**Annex to the description**

```
                              SEQUENCE LISTING


        (1) GENERAL INFORMATION


        (i) APPLICANT: SmithKline Beecham plc


        (ii) TITLE OF THE INVENTION:
                 Novel Compounds


        (iii) NUMBER OF SEQUENCES: 2


        (iv) CORRESPONDENCE ADDRESS:
          (A) ADDRESSEE: SmithKline Beecham
          (B) STREET: New Horizons Court
          (C) CITY: Brentford
          (D) STATE: Middlesex
          (E) COUNTRY: England
          (F) ZIP: TW8 9EP


        (v) COMPUTER READABLE FORM:
          (A) MEDIUM TYPE: Diskette
          (B) COMPUTER: IBM Compatible
          (C) OPERATING SYSTEM: DOS
          (D) SOFTWARE: FastSEQ for Windows Version 2.0


        (vi) CURRENT APPLICATION DATA:
          (A) APPLICATION NUMBER:
          (B) FILING DATE:
          (C) CLASSIFICATION:


        (vii) PRIOR APPLICATION DATA:
          (A) APPLICATION NUMBER:
          (B) FILING DATE:




        (viii) ATTORNEY/AGENT INFORMATION:
          (A) NAME:
          (B) REGISTRATION NUMBER:
          (C) REFERENCE/DOCKET NUMBER:  GP30015
```

(ix) TELECOMMUNICATION INFORMATION:

   (A) TELEPHONE:

   (B) TELEFAX:

   (C) TELEX:


   (2) INFORMATION FOR SEQ ID NO:1:


(i) SEQUENCE CHARACTERISTICS:

   (A) LENGTH: 285 base pairs

   (B) TYPE: nucleic acid

   (C) STRANDEDNESS: single

   (D) TOPOLOGY: linear


(ii) MOLECULE TYPE: cDNA


(xi) SEQUENCE DESCRIPTION: SEQ ID NO:1:


```
ATGGCTGCCG CATGCCGCTG CCTCTCCCTC CTGCTCCTGT CCACCTGTGT GGCTCTGTTG      60
CTGCAGCCAC TGCTGGGTGC CCGGGGAGCC CCGTTGGAGC CATTGTACCC AGGGGACAAT     120
ACCACACCGG AGCAGATGGC CCAGTACACA GCTGAGCTCC GTAGATACAT CAACATGCTG     180
ACCAGGCATA GGTATGGAGA AAGAGACAAA GAGGACACGC TGGCCTTCTC AGAGTGGGGG     240
TCTTCCCATG CTGCTGTCCC CAGAGAGCTC AGCCCGCTGG ACTTG                     285
```


   INFORMATION FOR SEQ ID NO:2:

    .

   (i) SEQUENCE CHARACTERISTICS:

   (A) LENGTH: 95 amino acids

   (B) TYPE: amino acid

   (C) STRANDEDNESS: single

   (D) TOPOLOGY: linear


(ii) MOLECULE TYPE: protein


(xi) SEQUENCE DESCRIPTION: SEQ ID NO:2:


```
Met Ala Ala Ala Cys Arg Cys Leu Ser Leu Leu Leu Leu Ser Thr Cys
 1               5                   10                  15
Val Ala Leu Leu Leu Gln Pro Leu Leu Gly Ala Arg Gly Ala Pro Leu
                20                  25                  30
```

```
Glu Pro Leu Tyr Pro Gly Asp Asn Thr Thr Pro Glu Gln Met Ala Gln
         35                  40                  45
Tyr Thr Ala Glu Leu Arg Arg Tyr Ile Asn Met Leu Thr Arg His Arg
     50                  55                  60
Tyr Gly Glu Arg Asp Lys Glu Asp Thr Leu Ala Phe Ser Glu Trp Gly
65                  70                  75                  80
Ser Ser His Ala Ala Val Pro Arg Glu Leu Ser Pro Leu Asp Leu
             85                  90                  95
```